# EUROPEAN PATENT SPECIFICATION

(11) **EP 1 790 380 B1**
(45) Date of publication and mention of the grant of the patent: **06.05.2009**
(21) Application number: 06256012.3
(22) Date of filing: 24.11.2006
(51) Int. Cl.: A61N 1/05

(54) **Microelectrode array and method for producing the same**
Mikroelektroden-Anordnung sowie Verfahren zu deren Herstellung
Ensemble de microélectrodes et méthode pour le produire

(30) Priority: 29.11.2005 US 288759
(43) Date of publication of application: 30.05.2007
(73) Proprietor: Nitinol Development Corporation, Fremont, California 94539 (US)
(72) Inventor: Krulevitch, Peter, Pleasanton, CA 94566 (US)
(74) Representative: Belcher, Simon James

(56) References cited:
- WO-A-20/04011083
- US-A- 5 720 099
- US-A1- 2003 097 165
- US-A1- 2004 147 992
- US-A1- 2004 238 819

## Description

The present invention relates to electrodes and more particularly to high-density microelectrode arrays. The invention provides a method of fabricating such a flexible, stretchable, and implantable microelectrode array as well as an implantable medical device that includes the inventive microelectrode array.

Microelectrode arrays are currently being developed for a broad range of applications including, for example, for use in various implantable medical devices. Implantable medical devices are defined herein as a physical article used in medical treatment that can be introduced into living tissue. Some examples of medical devices, which can contain microelectrode arrays, include, for example, cochlear implants, visual prostheses, neurostimulators, muscular stimulators, and deep brain stimulators.

A typical microelectrode array consists of multiple micron to mm scale electrodes with conducting traces and contact pads for interfacing to driving electronics. The conductive traces (or conductive wires or lines) are used to connect the electrodes of the array to the contact pads, which, in turn, are used to interface with the driving electronics of the medical device.

Many medical devices that are approved by the US Food and Drug Administration include microelectrode arrays that comprise bulk platinum (Pt) traces and electrodes embedded within a polymer body (or matrix), which are manually assembled using conventional (i.e., non-microfabrication) techniques well known in the art. The polymer body of such arrays is typically comprised of silicone or polyurethane.

Recent experimental medical devices take advantage of microfabrication techniques such as photolithographic patterning of metal films and electroplating of metal films to produce microelectrode arrays with smaller feature sizes and a greater number of electrodes than traditional microelectrode arrays. These prior art microelectrode arrays typically use silicon or a polyamide substrate, thin film Pt traces and thicker electrode plated Pt electrodes. Recently, microelectrode arrays with silicone substrates and stretchable thin film gold traces have been developed. Such arrays are disclosed, for example, in US-6878643, US-A-2003/0097166, US-A-2003/0097165, US-A-2004/0243204, US-A-2004/0238819 and US-A-2005/0030698.

US-A-2004/0147992 discusses an implantable medical assembly with at least one electrode. An undulated wire is connected to the electrode. The electrode and wire are embedded in a biocompatible film.

Problems exist with all the approaches mentioned above. For example, silicon and polyamide, while compatible with micromachining processes, are not sufficiently compliant to meet application needs, and electroplated platinum is susceptible to cracking and delamination due to large residual stresses. While the techniques disclosed in the documents mentioned above are promising, thin gold traces are not acceptable, and producing high quality thick Pt electrodes on silicone using standard deposition techniques is extremely challenging. Also, many of the prior art microelectrode array designs are not flexible and stretchable enough to be used with current implantable medical devices.

In view of the drawbacks mentioned above with fabrication of prior art microelectrode arrays, there is still a need for providing an alternative method of fabricating microelectrode arrays that are flexible, stretchable and can be implanted safely within living tissue.

The present invention provides an approach for fabricating a microelectrode array that combines micromachining techniques with methods used for producing metal stents. The method of the present invention utilizes materials that are compatible with micromachining processes, and the materials are sufficiently compliant to meet current needs for use as a component of an implantable medical device.

In accordance with the present invention, there is provided a method of forming a microelectrode array as defined in appended claim 1. A first implantable and biocompatible polymeric layer is formed on a surface of a handle substrate. The first polymeric layer is then cured providing a cured first polymeric layer on the handle substrate. A carrier substrate including a plurality of patterned conductive features comprising metallic contact pads, metallic traces and metallic electrodes is formed. In accordance with the present invention and within the array, a single metallic electrode is contacted to a single metallic contact pad by a single metallic trace. In some embodiments of the present invention, it is possible that there could be more than one electrode associated with a single contact pad.

Each of the metallic traces of the patterned conductive features are patterned to have a zigzag (or serpentine) configuration with substantially rounded corners similar to designs used for expandable stents to allow for stretching of the microelectrode array. The metallic traces having this zigzag pattern and substantially rounded corners provide an electrical contact between neighbouring metallic electrodes and metallic contact pads. The patterned conductive features are then transferred to the first polymeric layer using bonding techniques and at least the carrier substrate is removed at this point of the inventive process to expose the surface of the first polymeric layer including the patterned conductive features. In some embodiments of the present invention, the conductive traces are transferred to the first polymeric layer with bonding, and the traces are held in place when the second polymeric layer is applied.

A second polymeric layer, that is also implantable and biocompatible, is then formed on the bonded structure such that the patterned conductive features are encapsulated (i.e., surrounded or encased) within the polymeric layers. It is noted that the polymeric layers used in the present invention are insulating materials that are generally hydrophobic. The second polymeric layer may be pre-patterned prior to forming on the bonded structure or the second polymeric layer may be patterned after application to the bonded structure. The patterns formed into the second polymeric layer are typically vias (i.e., openings) that extend down to the first patterned conductive features exposing the metallic contact pads and metallic electrodes. The patterns also define the shape of the microelectrode array. The vias can be filled with a conductive material and contacts can be made with other elements or components of an implantable medical device.

The above steps can be repeated numerous times to create multiple layers of metal with alternating polymeric layers to produce multi-layer three-dimensional stacks with increased number of electrodes. After all the metal and polymeric layers are formed, the devices are sectioned and removed from the carrier substrate utilizing conventional techniques

In addition to the method described above, the present invention also provides a microelectrode array, as defined in appended claim 8, that is useful in implantable medical devices. The inventive microelectrode array includes at least first and second implantable and biocompatible polymeric layers in which a plurality of patterned conductive features including metallic contact pads, metallic traces and metallic electrodes is sandwiched between them, wherein each metallic trace has a zigzag pattern and substantially rounded corners.

In addition to the array, the present invention also provides an implantable medical device which comprises at least the microelectrode array of the present invention.

Embodiments of the invention will now be described by way of example with reference to the accompanying drawings, in which:
FIGS. 1A-1E are pictorial representations illustrating the basic processing steps of the present invention; FIGS. 1A-1B and 1D-1E are cross sectional views, while FIG. 1C is a top down view.
FIG. 2 is a pictorial representation (pseudo-3D) showing a basic microelectrode array structure of the present invention.

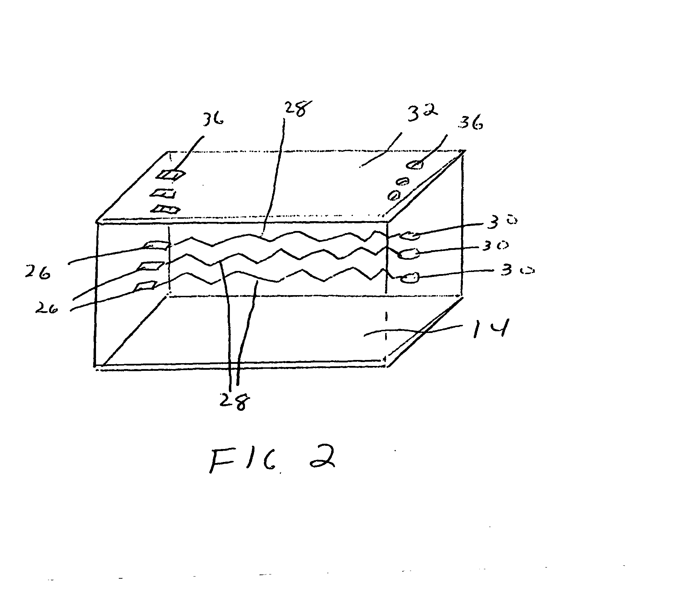

The drawings are provided for illustrative purposes and, as such, they are not drawn to scale. For example, in FIG. 2 the metal layer would be much thicker than that which is shown and the polymeric layers would be much thinner than that which is shown.

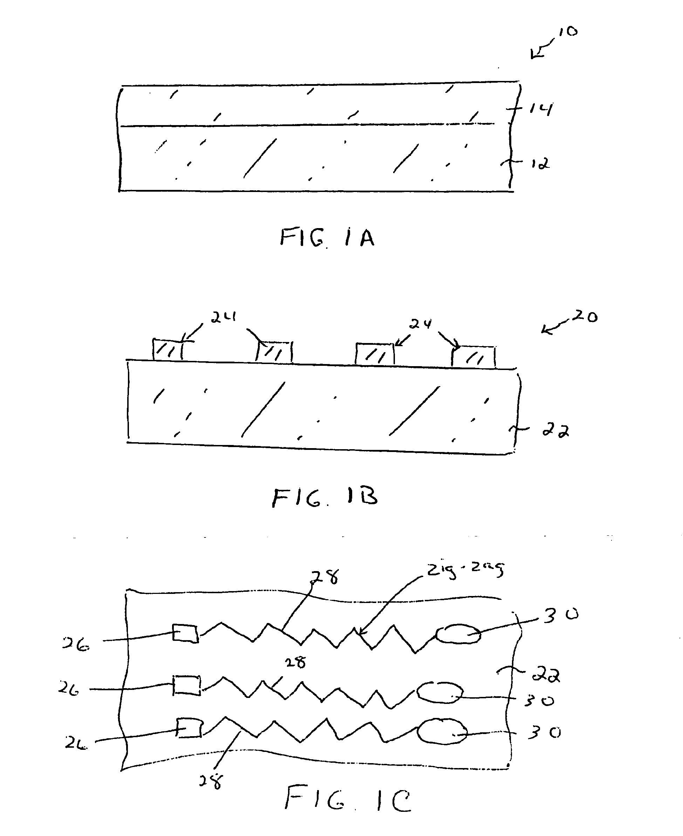

The method of the present invention begins with providing the two structures shown in FIGS. 1A or 1B. The two structures can be prepared in any order and, as such, the present invention is not limited to the order specified in the drawings. FIG. 1A shows a first structure 10 that includes a handle substrate 12 and a cured first implantable and biocompatible polymeric layer 14 located thereon. The handle substrate 12 may comprise a Si wafer, glass, plastic, ceramic or multilayers thereof. Typically, a Si wafer is used as the handle substrate 12 since they are flat, stable, routinely used in microfabrication applications and they are readily available. In some embodiments of the present invention, a non-stick layer (not shown) can be applied to the handle substrate 12 prior to forming the first polymeric layer 14 thereon.

The first polymeric layer 14 is applied to an upper exposed surface of the handle substrate 12 utilizing a conventional deposition process including, for example, spin-on coating, spray coating, dip-coating, casting, or vapour deposition (for parylene). Typically, a spin-on coating process is used to apply the first polymeric layer 14 to the handle substrate 12.

Notwithstanding the deposition technique used, the first polymeric layer 14 has an as-deposited thickness that is typically at least about 1 µm, preferably at least about 10 µm. The thickness will generally be not more than about 500 µm, preferably not more than to about 50 µm.

The first polymeric layer 14 is comprised of any implantable and biocompatible polymer. By "implantable" it is meant that the polymeric material can be inserted into a living site for medical usage. The term "biocompatible" denotes that the polymeric material is compatible with a living tissue or a living organism by not being toxic or injurious and by not causing immunological reaction. The polymeric material employed in the present invention is generally a hydrophobic material that is flexible and which can conform to many different shapes, including curved surfaces. It is noted that the term 'polymer' is used to denote a chemical compound with high molecular weight consisting of a number of structural units linked together by covalent bonds.

Illustrative examples of polymeric materials that can be used in the present invention as the first polymeric layer 14 include, but are not limited to: silicone polymers (i.e., organosiloxanes), polyurethanes, polyamides, parylene, fluoropolymers such as, for example, fluoropolymers such as polytetrafluoroethylene, polyolefins such as, for example, polyethylene and polypropylene, collagen, chitin, alginate, polyvinyl pyrrolidone, polyethylene glycol, polyethylene oxide, polyvinyl alcohol, polyglycol lactic acid, polylactic acid, polycaprolactone, polyamino acid, and a hydrogel such as, for example, carboxymethyl cellulose.

The first and second polymeric materials can be the same. The first and second polymeric materials can be different from one another.

In one preferred embodiment of the present invention, the first polymeric layer 14 is a silicone polymer. Silicone polymers generally are characterized as having the formula (RₙSiO_{4-n/2}) wherein R is an organic group, n is 1-3, and m is greater than or equal to 2. A silicone polymer contains a repeating silicon-oxygen backbone and has organic groups R attached to a significant proportion of the Si atoms by silicon-carbon bonds. In many of the commercially available silicones, most of the R groups can be an alkyl containing from 1 to about 20 carbon atoms, fluoroalkyl, phenyl, vinyl, and some of the remaining R groups can be hydrogen, chlorine, alkoxy, acyloxy or alkylamine.

In this preferred embodiment of the present invention, the first polymeric layer 14 includes one of poly(dimethylsiloxane), polyurethane, parylene, and the like. Of the various polymers mentioned above, poly(dimethylsiloxane) (PDMS) is highly preferred in the present invention. PDMS has low water permeability and protects electronic components from the environment. Also, PDMS is very flexible and will conform to curved surfaces. Additionally, PDMS is transparent, stretchable, resinous, rubbery and provides numerous applications for the microelectrode array of the present invention.

Curing of the as-deposited first polymeric layer 14 is performed at a temperature from about 20° to about 100°C for a time period from about 0.5 to about 48 hours. The curing temperature and time will vary depending on the material of the first polymeric layer 14 as well as the thickness of the as-deposited layer. Typically, and for PDMS having a thickness within the above range, a curing temperature of about 66°C for a time period from about 24 to 48 hours is employed. As is known to those skilled in the art, curing polymerizes the polymer.

FIG. 1B shows a second structure 20 that includes a carrier substrate 22 having a plurality of conductive features 24 located on a surface thereof. The carrier substrate 22 may comprise the same or different material as that of the handle substrate 12.

The term "conductive features" is used throughout this application to denote metallic electrodes, metallic traces, and metallic contact pads. In accordance with the present invention, the metallic traces provide electrical contact between neighbouring metallic pads and metallic electrodes. FIG. 1C shows a top down view showing a plurality of metallic electrodes 30, metallic traces 28 and metallic contact pads 26 arranged in the manner indicated above. It should be noted that more than one metallic electrode 30 can be associated with a single metallic contact pad 26.

In accordance with the present invention, the plurality of conductive features 24 is formed such that each of the metallic traces 28 has a zigzag (or serpentine) pattern and substantially rounded corners. This design for the metallic traces is similar to those found in many medical stents and it also allows for stretching of the inventive microelectrode array. That is, each of the metallic traces 28 present in the inventive microelectrode array is arranged such that it has sharp turns and angles that alter the course of the metallic trace. The number of turns and angles present in each metallic trace 28 may vary depending on the total area of the final device. Each metallic trace 28 must, however, have at least one turn and angle that changes the course of the metallic trace connected a metallic contact pad to a metallic electrode. Typically, each metallic trace 28 is designed to contain from about 2 to about 200 turns and angles, depending on the length of the device. The term "substantially rounded" is used herein to denote a radius of curvature greater than approximately the width of the trace.

The term "metallic" is used in the present invention to denote a material that includes at least one metal or metal alloy that is conductive. Illustrative examples of metallic materials that can be used in forming the plurality of conductive features 24 include, but are not limited to: Pt, Ti and alloys such as an alloy ofNiTi. In one embodiment of the present invention, Pt is used as the metallic material of the least one conductive feature 24. In another embodiment of the present invention, an alloy of NiTi known as Nitinol available from Nitinol Devices & Components Inc can be used since this alloy is superelastic and thus can provide metallic traces 28 that are capable of exhibiting extremely large deformations. It is noted that in the present invention a single metallic foil or sheet can be used to provide the plurality of conductive features 24 which is an advantage over some of the prior art in which multiple films are used in creating such features.

The plurality of conductive features 24 can be formed utilizing laser machining in which a metallic foil or sheet is first applied to a surface of the carrier substrate 22. The foil or sheet can be patterned by laser cutting (like stents) or wet chemical etching.

The thickness of metallic foil or sheet formed at this point of the present invention may vary and can be determined by the skilled artisan. Typically, the metallic foil or sheet formed at this point of the present invention has thickness from about 5 to about 500 microns, with a thickness from about 10 to about 75 microns being more typical.

After applying the metallic foil or sheet to the surface of the carrier substrate 22, the plurality of conductive features 24 is formed by laser machining. Laser machining is generally a technique that is used in fabricating medical stents and is thus well known in medical device fabrication. Typically, laser machining is performed utilizing a laser system that is scanned over the substrate, ablating material when the laser energy contacts the substrate.

In another embodiment of the present invention, the plurality of conductive features 24 is formed utilizing photolithography and etching. The term "photolithography" is used throughout this application to denote a patterning technique in which a photoresist (either positive-tone or negative-tone) is applied to the upper exposed surface of a film needing patterning. The photoresist can be applied by utilizing any deposition technique, with spin-on coating, dip-coating, and spray coating being highly preferred. Following the application of the photoresist, the photoresist is exposed to a pattern of radiation. In the present invention, the pattern of radiation allows for the formation of the plurality of conductive features 24. After radiation exposure, the exposed resist is developed utilizing a conventional resist developer. The lithographic step thus forms a patterned photoresist having the pattern of the plurality of conductive features 24 located therein. Because of the nature of the photolithographic process the pattern formed into the resist has inherent corner rounding. This pattern is then transferred to the metallic film utilizing an etching process. The etching process may include a dry etching technique such as, for example, reactive-ion etching (RIE), ion beam etching, plasma etching or laser ablation. Alternatively, the etching can be achieved utilizing a chemical wet etching process in which a chemical etchant that selectively removes the exposed portions of the metallic film is used. After pattern transfer via etching, the patterned photoresist is removed utilizing a conventional stripping process well known to those skilled in the art.

In some embodiments of the present invention, the conductive features are transferred to the first polymeric layer and the conductive features are held in place, while the second polymeric layer is applied.

After providing the structures shown in FIGS. 1A and 1B, those two structures (10 and 20) are brought into intimate contact with each other such that the plurality of conductive features 24 will be transferred to the surface of the first polymeric layer 14. Next, the contacted structures are bonded together. Bonding, which can be achieved in the presence or absence of an applied external force, is performed utilizing a nominal room temperature bonding process. By "nominal room temperature" it is meant a bonding temperature from about 20°C to about 40°C is used. The bonding can be performed in air, under vacuum, or in an inert gas ambient.

In some embodiments of the present invention, the first polymeric layer 14 on the surface of the handle substrate 12 is treated prior to bonding to activate the surface of the first polymeric layer 14. When this treatment is performed, the structure shown in FIG. 1A is subjected to an oxygen plasma that activates the polymeric surface and promotes the adhesive of the plurality of conductive features 24 to the first polymeric layer 14. The oxygen plasma treatment is performed at a radio frequency (RF) power from about 75 to about 200 Watts using an oxygen flow from about 25 to about 100 sccm. The plasma treatment is performed for a time period from about 5 to about 10 minutes.

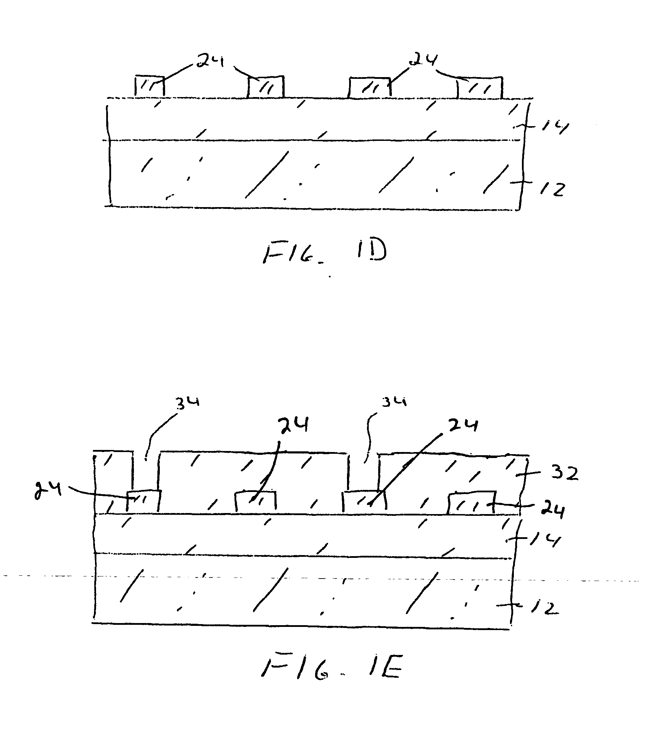

After bonding the two structures together, at least the carrier substrate 22 is removed by peeling off the bonded components. In some embodiments, the bonded structure can be removed from the carrier substrate 22 by utilizing a conventional lift-off procedure. The resultant structure after bonding and removal of the carrier substrate 22 is shown in FIG. 1D.

FIG. 1E shows the structure after forming a second implantable and biocompatible polymeric layer 32 on the bonded structure such that the plurality of conductive features 24 is surrounded, i.e., encased, within the two polymeric layers. The second implantable and biocompatible polymeric layer 32 may be comprised of the same or different, preferably the same, polymeric material as that of the first polymeric layer 14. In a highly preferred embodiment, polymeric layers 14 and 32 are both silicone polymers, with PDMS being most preferred. As shown, the second polymeric layer 32 includes a plurality of vias 34 which extend down through the second polymeric layer 32 and provide contact openings where a conductive material can be formed. The vias 34 expose some of the underlying conductive features 24, for example the metallic pads and the metallic electrodes 30. Thus, the vias 34 are formed in preselected locations within the inventive structure.

The structure shown in FIG. 1E can be formed by first applying a blanket layer of the second polymeric layer 32 to the structure shown, for example, in FIG. 1D. The blanket layer of the second polymeric layer 32 can be deposited utilizing one of the above mentioned deposition processes that was used in forming the first polymeric layer 14. The vias 34 are formed into the second polymeric layer 32 by photolithography and etching. In some embodiments of the present invention and prior to forming the photoresist on the surface of the second polymeric layer 32, the second polymeric layer 32 may be subjected to an oxygen plasma treatment which allows the resist to wet the polymeric surface preventing beading and ensuring formation of smooth and uniform resist coating on the second polymeric layer 32.

In an alternative embodiment, the structure shown in FIG. 1E is formed by providing a pre-patterned second polymeric layer 32 that contains said vias on a carrier substrate. This pre-patterned structure is formed by first applying the second polymeric layer 32 to a carrier substrate, subjecting the second polymeric layer 32 to photolithography and etching. This structure is then bonded to the structure shown in FIG. 1D utilizing the bonding conditions mentioned above. The blanket layer of second polymeric material can be subjected to oxygen plasma prior to photoresist application and a second treatment with oxygen plasma may occur after patterning the vias therein.

A conductive material 36 is then filled into the vias 34 utilizing a conventional deposition process and following deposition any conductive material outside the vias can be removed utilizing a conventional planarization process. The filled vias allow for the inventive microelectrode array shown in FIG. 2 to be interfaced with other components of the implantable medical devices including, for example, an energy source and a sensor. It is noted that the sidewalls of the vias provide openings to make contact to the electrodes.

The above steps of the present invention can be repeated numerous times to create multiple layers of metal with alternating polymeric layers to produce multi-layer three-dimensional stacks with increased number of electrodes. After all the metal and polymeric layers are formed, the devices are sectioned and removed from the carrier substrate utilizing conventional techniques well known in the art.

As stated above, the inventive microelectrode array is suitable for use as a component in an implantable medical device. Such implantable medical devices include, for example, cochlear implants, visual prostheses, neurostimulators, muscular stimulators, and deep brain stimulators. Although the inventive microelectrode array is specifically mentioned to be suitable for use in an implantable medical device, it can also find uses in electronic devices other than implantable medical devices. Other applications for the inventive microelectrode array include, but are not limited to: electrodes and electrical interconnects for medical devices that are not implanted, consumer electronics subjected to water immersion or splashing, and underwater sensing systems.

It is observed that the method of the present invention has several advantages over prior art techniques used in forming microelectrode arrays. First, the inventive method fabricates a microelectrode array with relatively thick conductive features that are flexible, stretchable and rugged. Moreover, the metallic pads, traces, and electrodes are made using a single continuous metallic sheet or foil simplifying the overall process and eliminating potential problems associated with depositing Pt or another conductive metal or a separate metal film. The method of the invention is simple and low cost, and enables the fabrication of microelectrode arrays with to many electrodes (for example hundreds of electrodes or thousands of electrodes). In addition, the method takes advantage of well-characterized manufacturing techniques (such as, for example, laser machining of stents and photolithography) and lends itself well to mass production.

## Claims

1. A method of forming a microelectrode array for use as an element in implantable medical device comprising:
(a) bonding a first structure (10) comprising at least a first implantable and biocompatible polymeric layer (14) and a second structure (20) comprising a plurality of conductive features (24) including metallic contact pads (26), metallic traces (28), and metallic electrodes (30), wherein each metallic trace (28) has a zigzag pattern and substantially rounded comers; and
(b) forming a second implantable and biocompatible polymeric layer (32) to said bonded structure, said second polymeric layer (32) covering said plurality of conductive features (24) and has vias (34) therein that extend down to said metallic contact pads (26) and said metallic electrodes (30); **characterised in that** said steps of bonding and forming are repeated numerous times to create multiple layers of metal with alternating polymeric layers to form a multi-layered 3D microelectrode array.

2. The method of Claim 1 wherein at least one of the first and second polymeric layers (14, 32) comprises one or more of a silicone polymer, a polyurethane, a polyamide, parylene, a fluoropolymer, a polyolefin, collagen, chitin, alginate, polyvinyl pyrrolidone, polyethylene glycol, polyethylene oxide, polyvinyl alcohol, polyglycol lactic acid, polylactic acid, polycaprolactone, polyamino acid, and a hydrogel.

3. The method of Claim 2 wherein both said first and second polymeric layers (14, 32) are comprised of a silicone polymer, preferably poly(dimethylsiloxane).

4. The method of Claim 1 wherein said plurality of conductive features (24) are comprised of a conductive metal or metal alloy which comprises at least one of Pt, Ti and NiTi.

5. The method of Claim 1 wherein said providing said bonded substrate comprises a nominal room temperature bonding process and contacting of said first structure (10) to said second structure (20) such that an exposed surface of said first polymeric layer (14) is in contact with an exposed surface of said plurality of conductive features (24).

6. The method of Claim 1 wherein said plurality of conductive features (24) is formed by laser etching a metallic sheet or foil or photolithography and etching of a metallic sheet or foil.

7. The method of Claim 1 further comprising forming a conductive material within said vias (34).

8. A microelectrode array for use as an element in an implantable medical device comprising first and second implantable and biocompatible polymeric layers (14, 32) in which a plurality of patterned conductive features (24) including metallic contact pads (26), metallic traces (28) and metallic electrodes (30) is sandwiched therebetween, wherein each metallic trace (28) has a zigzag pattern and substantially rounded corners, wherein the microelectrode array further comprises additional implantable and biocompatible polymeric layers atop the second polymeric layer, (32) **characterised in that** a plurality of conductive features (24) is present between each polymeric layer.

9. The microelectrode array of Claim 8 wherein at least one of the first and second polymeric layers (14, 32) comprises one or more of a silicone polymer, a polyurethane, a polyamide, parylene, a fluoropolymer, a polyolefin, collagen, chitin, alginate, polyvinyl pyrrolidone, polyethylene glycol, polyethylene oxide, polyvinyl alcohol, polyglycol lactic acid, polylactic acid, polycaprolactone, polyamino acid, and a hydrogel.

10. The microelectrode array of Claim 9 wherein said silicone polymer is poly(dimethylsiloxane).

11. The microelectrode array of Claim 8 wherein said plurality of conductive features (24) are comprised of a conductive metal or metal alloy which comprises at least one of Pt, Ti and NiTi.

12. The microelectrode array of Claim 8 further comprising a plurality of conductively filled vias (34) in said second polymeric layer (32) that expose said metallic contact pads (26) and said metallic electrodes (30).

13. The microelectrode array of Claim 8 wherein said zigzag pattern contains from about 2 to about 200 turns and angles therein.

14. An implantable medical device comprising a microelectrode array according to claim 8, and wherein said second polymeric layer (32) has conductively filled vias (34) that extend down to said metallic contact pad (26) and said metallic electrode (30).

## Patentansprüche

1. Verfahren zum Erzeugen einer Mikroelektrodenanordnung zur Verwendung als ein Element in einer implantierbaren medizinischen Vorrichtung, das aufweist:
(a) Bonden einer ersten Struktur (10), die wenigstens eine erste implantierbare und biokompatible Polymerschicht (14) aufweist, und einer zweiten Struktur (20), die eine Vielzahl von leitenden Merkmalen (24) aufweist, welche metallische Kontaktflächen (26), metallische Spuren (38) und metallische Elektroden (30) umfassen, wobei jede metallische Spur (28) ein Zickzackmuster und im Wesentlichen abgerundete Ecken hat; und
(b) Erzeugen einer zweiten implantierbaren und biokompatiblen Polymerschicht (32) an der gebondeten Struktur, wobei die zweite Polymerschicht (32) die Vielzahl der leitenden Merkmale (24) überdeckt und Kontaktlöcher (34) umfasst, die sich nach unten zu den metallischen Kontaktflächen (26) und den metallischen Elektroden (30) erstrecken; **dadurch gekennzeichnet, dass** die Schritte des Bondens und des Erzeugens mehrmals wiederholt werden, um mehrere Schichten aus Metall abwechselnd mit Polymerschichten zu erzeugen, um einen mehrschichtige 3D-Mikroelektrodenanordnung zu bilden.

2. Verfahren nach Anspruch 1, bei dem wenigstens eine, die erste oder die zweite Polymerschicht (14, 32), eines oder mehrere aus einem Silikonpolymer, einem Polyurethan, einem Polyamid, Parylen, einem Fluorpolymer, einem Polyolefin, Kollagen, Chitin, Alginat, Polyvinylpyrrolidon, Polyethylenglykol, Polyethylenoxid, Polyvinylalkohol, Polyglykolmilchsäure, Polymilchsäure, Polycaprolacton, Polyaminsäure und einem Hydrogel aufweist.

3. Verfahren nach Anspruch 2, bei dem sowohl die erste als auch die zweite Polymerschicht (14, 32) aus einem Silikonpolymer, bevorzugt Polydimethylsiloxan, bestehen.

4. Verfahren nach Anspruch 1, bei dem die Vielzahl der leitenden Merkmale (24) aus einem leitenden Metall oder einer metallischen Legierung, die wenigstens eines aus Pt, Ti und NiTi aufweist, besteht.

5. Verfahren nach Anspruch 1, bei dem das Bereitstellen des gebondeten Substrates einen Bondingprozess bei nominaler Raumtemperatur und das Inkontaktbringen der ersten Struktur (10) mit der zweiten Struktur (20) derart, dass eine freiliegende Fläche der ersten Polymerschicht (14) in Kontakt mit einer freiliegenden Fläche der Vielzahl der leitenden Merkmale (24) ist, aufweist.

6. Verfahren nach Anspruch 1, bei dem die Vielzahl der leitenden Merkmale (24) durch Laserätzen einer metallischen Lage oder Folie oder durch Fotolitografie und Ätzen einer metallischen Lage oder Folie gebildet wird.

7. Verfahren nach Anspruch 1, das weiter das Formen eines leitenden Materials innerhalb der Kontaktlöcher (34) aufweist.

8. Mikroelektrodenanordnung zur Verwendung als ein Element in einer implantierbaren medizinischen Vorrichtung, die eine erste und eine zweite implantierbare und biokompatible Polymerschicht (14, 32) aufweist, zwischen denen eine Vielzahl strukturierter leitender Merkmale (24), die metallische Kontaktflächen (26), metallische Spuren (28) und metallische Elektroden (30) umfassen, eingeschlossen ist, wobei jede metallische Spur (28) ein Zickzackmuster und im Wesentlichen gerundete Ecken hat, wobei die Mikroelektrodenanordnung weiter zusätzliche implantierbare und biokompatible Polymerschichten auf der zweiten Polymerschicht (32) aufweist, **dadurch gekennzeichnet, dass** zwischen allen Polymerschichten eine Vielzahl leitender Merkmale (24) vorhanden ist.

9. Mikroelektrodenanordnung nach Anspruch 8, bei der wenigstens eine, die erste oder die zweite Polymerschicht (14, 32), eines oder mehrere aus einem Silikonpolymer, einem Polyurethan, einem Polyamid, Parylen, einem Fluorpolymer, einem Polyolefin, Kollagen, Chitin, Alginat, Polyvinylpyrrolidon, Polyethylenglykol, Polyethylenoxid, Polyvinylalkohol, Polyglykolmilchsäure, Polymilchsäure, Polycaprolacton, Polyaminsäure und einem Hydrogel aufweist.

10. Mikroelektrodenanordnung nach Anspruch 9, bei der das Silikonpolymer Polydimethylsiloxan ist.

11. Mikroelektrodenanordnung nach Anspruch 8, bei der die Vielzahl der leitenden Merkmale (24) aus einem leitenden Metall oder einer metallischen Legierung, die wenigstens eines aus Pt, Ti und NiTi aufweist, besteht.

12. Mikroelektrodenanordnung nach Anspruch 8, die weiter eine Vielzahl leitend gefüllter Kontaktlöcher (34) in der zweiten Polymerschicht (32) aufweist, die die metallischen Kontaktflächen (26) und die metallischen Elektroden (30) freilegen.

13. Mikroelektrodenanordnung nach Anspruch 8, bei der das Zickzackmuster ungefähr 2 bis ungefähr 200 Kehren und Winkel enthält.

14. Implantierbare medizinische Vorrichtung, die eine Mikroelektrodenanordnung nach Anspruch 8 aufweist, und bei der die zweite Polymerschicht (32) leitend gefüllte Kontaktlöcher (34) hat, die sich hinab zu der metallischen Kontaktfläche (26) und der metallischen Elektrode (30) erstrecken.

## Revendications

1. Procédé de formation d'un réseau de microélectrodes pour une utilisation en tant qu'élément dans un dispositif médical implantable comprenant les étapes consistant à :
(a) lier une première structure (10) comprenant au moins une première couche polymérique implantable et biocompatible (14) et une deuxième structure (20) comprenant une pluralité de caractéristiques conductrices (24) comprenant des pastilles de contact métalliques (26), des pistes métalliques (28), et des électrodes métalliques (30), dans lequel chaque piste métallique (28) a un motif en zigzag et des coins sensiblement arrondis ; et
(b) former une deuxième couche polymérique implantable et biocompatible (32) sur ladite structure liée, ladite deuxième couche polymérique (32) recouvrant ladite pluralité de caractéristiques conductrices (24) et comportant des trous d'interconnexion (34) dans celle-ci qui s'étendent jusqu'auxdites pastilles de contact métalliques (26) et jusqu'auxdites électrodes métalliques (30) ; **caractérisé en ce que** lesdites étapes de liaison et de formation sont répétées de nombreuses fois pour créer de multiples couches de métal avec des couches polymériques alternées pour former un réseau de microélectrodes 3D à multiples couches.

2. Procédé selon la revendication 1, dans lequel au moins l'une des première et deuxième couches polymériques (14, 32) comprend un ou plusieurs d'un polymère de silicone, d'un polyuréthane, d'un polyamide, d'un parylène, d'un fluoropolymère, d'un polyoléfine, d'un collagène, d'une chitine, d'un alginate, d'un polyvinylpyrrolidone, d'un polyéthylène glycol, d'un polyéthylène-oxyde, d'un alcool polyvinylique, d'un acide lactique de polyglycol, d'un acide polylactique, d'un polycaprolactone, d'un polyaminoacide, et d'un hydrogel.

3. Procédé selon la revendication 2, dans lequel lesdites première et deuxième couches polymériques (14, 32) sont toutes deux composées d'un polymère de silicone, de préférence un poly-(diméthylsiloxane).

4. Procédé selon la revendication 1, dans lequel ladite pluralité de caractéristiques conductrices (24) sont composées d'un métal ou d'un alliage métallique conducteur qui comprend au moins l'un du Pt, du Ti et du NiTi.

5. Procédé selon la revendication 1, dans lequel ladite réalisation dudit substrat lié comprend un processus de liaison à température ambiante nominale et la mise en contact de ladite première structure (10) avec ladite deuxième structure (20) de sorte qu'une surface exposée de ladite première couche polymérique (14) soit en contact avec une surface exposée de ladite pluralité de caractéristiques conductrices (24).

6. Procédé selon la revendication 1, dans lequel ladite pluralité de caractéristiques conductrices (24) est formée par gravure au laser d'une feuille ou d'une mince feuille de métal ou par photolithographie et gravure d'une feuille ou d'une mince feuille de métal.

7. Procédé selon la revendication 1, comprenant en outre la formation d'un matériau conducteur dans lesdits trous d'interconnexion (34).

8. Réseau de microélectrodes pour une utilisation en tant qu'élément dans un dispositif médical implantable comprenant des première et deuxième couches polymériques implantables et biocompatibles (14, 32) dans lesquelles une pluralité de caractéristiques conductrices dessinées (24) comprenant des pastilles de contact métalliques (26), des pistes métalliques (28) et des électrodes métalliques (30) sont intercalées entre elles, dans lequel chaque piste métallique (28) a un motif en zigzag et des coins sensiblement arrondis, dans lequel le réseau de microélectrodes comprend en outre des couches polymériques implantables et biocompatibles supplémentaires au dessus de la deuxième couche polymérique (32), **caractérisé en ce qu'**une pluralité de caractéristiques conductrices (24) est présente entre chaque couche polymérique.

9. Réseau de microélectrodes selon la revendication 8, dans lequel au moins l'une des première et deuxième couches polymériques (14, 32) comprend un ou plusieurs d'un polymère de silicone, d'un polyuréthane, d'un polyamide, d'un parylène, d'un fluoropolymère, d'un polyoléfine, d'un collagène, d'une chitine, d'un alginate, d'un polyvinylpyrrolidone, d'un polyéthylène glycol, d'un polyéthylène-oxyde, d'un alcool polyvinylique, d'un acide lactique de polyglycol, d'un acide polylactique, d'un polycaprolactone, d'un polyaminoacide, et d'un hydrogel.

10. Réseau de microélectrodes selon la revendication 9, dans lequel ledit polymère de silicone est du poly(diméthylsiloxane).

11. Réseau de microélectrodes selon la revendication 8, dans lequel ladite pluralité de caractéristiques conductrices (24) est composée d'un métal ou d'un alliage métallique conducteur qui comprend au moins l'un du Pt, du Ti et du NiTi.

12. Réseau de microélectrodes selon la revendication 8, comprenant en outre une pluralité de trous d'interconnexion remplis d'un conducteur (34) dans ladite deuxième couche polymérique (32) qui exposent lesdites pastilles de contact métalliques (26) et lesdites électrodes métalliques (30).

13. Réseau de microélectrodes selon la revendication 8, dans lequel ledit motif en zigzag contient d'environ 2 à environ 200 tours et angles dans celui-ci.

14. Dispositif médical implantable comprenant un réseau de microélectrodes selon la revendication 8, et dans lequel ladite deuxième couche polymérique (32) comporte des trous traversants remplis d'un conducteur (34) qui s'étendent jusqu'à ladite pastille de contact métallique (26) et jusqu'à ladite électrode métallique (30).
